# EUROPEAN PATENT APPLICATION

(11) **EP 4 729 608 A1**
(43) Date of publication of application: **22.04.2026**
(21) Application number: 25706272.9
(22) Date of filing: 17.02.2025
(51) Int. Cl.: C12N 5/071, A61K 35/44, A61P 9/10

(54) **UMBILICAL CORD BLOOD-DERIVED ENDOTHELIAL PROGENITOR CELLS, AND COMPOSITION FOR PREVENTING OR TREATING ISCHEMIC DISEASES COMPRISING SAME**

(30) Priority: 16.08.2024 KR 20240110016
(71) Applicant: Youth Bio Global Co., Ltd., Seoul 08381 (KR)
(72) Inventor: YOO, Seung Ho, Seoul 05555 (KR); CHANG, Chi Young, Seoul 08223 (KR)
(74) Representative: dompatent
(86) International application number: PCT/KR2025/002296
(87) International publication number: WO 2026/038636

(57) **Abstract**

The present disclosure relates to a cord blood-derived vascular endothelial progenitor cell and a composition for preventing or treating ischemic diseases containing the same, and more specifically, to a highly pure cord blood-derived vascular endothelial progenitor cell exhibiting specific surface antigen properties, a method for obtaining a cord blood-derived vascular endothelial progenitor cell through preprocessing of cord blood, and a composition for preventing or treating ischemic disease containing the cord blood-derived vascular endothelial progenitor cell.

The high-purity cord blood-derived vascular endothelial progenitor cell of the present disclosure exhibits colony formation and high proliferation ability and angiogenic potential in the body, and thus can exhibit excellent effects in the prevention or treatment of various ischemic diseases caused by vascular contraction or occlusion.

## Description

### [TECHNICAL FIELD]

The present disclosure relates to a cord blood-derived vascular endothelial progenitor cell and a composition for preventing or treating ischemic diseases containing the same, and more specifically, to a highly pure cord blood-derived vascular endothelial progenitor cell exhibiting specific surface antigen properties, a method for obtaining a cord blood-derived vascular endothelial progenitor cell through preprocessing of cord blood, and a composition for preventing or treating ischemic disease containing the cord blood-derived vascular endothelial progenitor cell.

### [BACKGROUND ART]

Ischemic diseases such as heart disease, cerebrovascular disease, and peripheral vascular disease have high morbidity and mortality rates, and it is often difficult to obtain sufficient effects with existing internal medicine, interventional treatment, and surgical treatment. For this reason, a new treatment method that promotes angiogenesis and improves blood flow in ischemic tissue is needed.

Endothelial progenitor cells (EPC) have the ability to promote angiogenesis, and these cells can be derived from bone marrow, peripheral blood, and cord blood. Particularly, cord blood is evaluated as a suitable resource for cell therapy because it is easy to obtain, has little immune rejection, and has relatively few ethical issues. Endothelial progenitor cells express vascular endothelial cell markers such as CD34, CD144, and CD184, and these cells migrate to damaged blood vessel sites and help vascular regeneration.

Previous studies have proposed various cell therapies using vascular endothelial progenitor cells, but most of the studies have problems with cell purity and proliferation, and have difficulties in clinical application due to lack of optimization of culture conditions, growth factors, and attachment substrates used in the cell acquisition process.

Meanwhile, the half-life of heparin in blood is within 1 to 2 hours in the human body, and it is reported that heparin loses its activity rapidly by binding to numerous proteins present in the blood (Semin Intervent Radiol 2010 Dec; 27(4): 360-367). The binding and dissociation of heparin to blood cytokines and growth factors (J Cell Mol Med. 2018. PMID: 30334335) proteins is affected by temperature (Biochem Physiol. 2018.), and it has also been reported that the interaction with these proteins can affect the cellular function of blood ECFCs or late proliferating endothelial progenitor cells. Therefore, it can be identified that the number of proteins that can bind heparin is considerable, and the result of the binding of heparin to proteins has a very complex correlation (Stem Cell Res 2014 May;12(3):703-15) in terms of the cellular effects.

### [DETAILED DESCRIPTION OF THE INVENTION]

### [TECHINICAL PROBLEM]

Accordingly, the inventors of the present disclosure conduct research and effort to obtain a high-purity vascular endothelial progenitor cell that can be applied clinically, and as a result, by treating cord blood with heparin to isolate and obtain a monocyte and culturing them under a specific condition, a high-purity vascular endothelial progenitor cell exhibiting specific surface antigen properties can be obtained, thereby completing the present disclosure.

The purpose of the present disclosure is to provide a cord blood-derived vascular endothelial progenitor cell, a method for obtaining the same, and a composition for preventing or treating ischemic disease containing the cord blood-derived vascular endothelial progenitor cell.

### [TECHNICAL SOLUTION]

In an aspect of the present disclosure, a cord blood-derived vascular endothelial progenitor cell is provided, wherein at least 50% or more of the cell population expresses CD34 marker, and wherein at least 5% or less of the cell population expresses CD90 marker.

In the cord blood-derived vascular endothelial progenitor cell, at least 80% or more of the cell population may express the CD34 marker.

In the cord blood-derived vascular endothelial progenitor cell, at least 80% or more of the cell population may express CD144 marker and the CD184 marker.

The cord blood-derived vascular endothelial progenitor cell may be cultured for at least 10 passages.

The cord blood-derived vascular endothelial progenitor cell may increase an expression of at least one angiogenesis-stimulating factor selected from ANGPT2, MCP-1, MMP-1, or PIGF.

In another aspect of the present disclosure, a cell therapy composition for the prevention or treatment of ischemic disease is provided, including the cord blood-derived vascular endothelial progenitor cell as an effective ingredient.

In still another aspect of the present disclosure, a method for obtaining a cord blood-derived vascular endothelial progenitor cell may include:
treating cord blood with heparin to isolate and obtain monocytes from the cord blood; and
culturing the monocytes in a medium containing fucoidan, oleuropein, and vascular endothelial growth factor as effective ingredients to obtain cord blood-derived vascular endothelial progenitor cell.

The heparin may be treated at a concentration of 1 to 1000 U per 1 ml of cord blood.

The heparin treatment may be performed at 1 to 20° C for 1 to 8 hours.

The method may further include passaging the cord blood-derived vascular endothelial progenitor cell expressing CD34 marker among the obtained cord blood-derived vascular endothelial progenitor cells to finally obtain a high-purity umbilical cord blood-derived vascular endothelial progenitor cell.

### [ADVANTAGEOUS EFFECTS OF THE INVENTION]

The high-purity cord blood-derived vascular endothelial progenitor cell of the present disclosure exhibits colony formation and high proliferation ability and angiogenic potential in the body, and thus can exhibit excellent effects in the prevention or treatment of various ischemic diseases caused by vascular contraction or occlusion.

In addition, the present disclosure can secure a sufficient number of colonies of a cord blood-derived vascular endothelial progenitor cell through heparin treatment under specific conditions, and thus can contribute to the mass production of cell therapy agents, and the like.

### [DESCRIPTION OF THE DRAWINGS]

FIG. 1 is a graph comparing the number of colonies obtained after treating cord blood with the same concentration of heparin and CPDA-1 and going through the same culture process.
FIG. 2 is a graph comparing the number of colonies obtained after treating cord blood 3 months after starting to use heparin and going through the same culture process.
FIG. 3 is a result of analyzing the surface antigen properties of a cord blood-derived vascular endothelial progenitor cell of the present disclosure.
FIG. 4 shows a photograph of colonies attached to fibronectin recovered with a Triple Solution product, separated from cells, and then cultured.
FIG. 5 is a graph measuring blood flow and new capillary density after treating a mouse with a cord blood-derived vascular endothelial progenitor cell of the present disclosure.

### [BEST MODE]

Hereinafter, the present disclosure will be described in detail. Prior to this, the term or word used in this specification and claim should not be interpreted as limited to the usual or dictionary meaning, and should be interpreted as meaning and concept that conforms to the technical idea of the present disclosure based on the principle that the inventor can appropriately define the concept of the term to explain his or her own invention in the best way. Therefore, the configuration described in the embodiment described in this specification is only the most preferred embodiment of the present disclosure and do not represent all of the technical ideas of the present disclosure. Therefore, it should be understood that there may be various equivalents and modified examples that can replace them at the time of the present application.

The cord blood-derived vascular endothelial progenitor cell of the present disclosure is characterized in that, in terms of an undifferentiated stem cell-related marker, at least 50% or more of the cell population expresses CD34 marker, and at least 5% or less of the cell population expresses CD90 marker.

Preferably, the cord blood-derived vascular endothelial progenitor cell may express the CD34 marker in at least 80% or more of the cell population.

In addition, the cord blood-derived vascular endothelial progenitor cell may express CD144 marker and CD184 marker in at least 80% or more of the cell population.

The cord blood-derived vascular endothelial progenitor cell may be cultured for at least 10 passages or more, and may exhibit the above property within 12 days after acquisition from isolated monocytes.

The cord blood-derived vascular endothelial progenitor cell may increase the expression of one or more angiogenesis stimulating factors selected from ANGPT2, MCP-1, MMP-1, and PIGF. Due to the increase in the expression of the angiogenesis stimulating factors, vascular regeneration, vascular recovery, and vascular differentiation related to the angiogenesis process, including endothelial cell activation, migration, proliferation, matrix remodeling, and cell stabilization, may be induced. In one embodiment of the present disclosure, the expression amount of the angiogenesis stimulating factors of the cord blood-derived vascular endothelial progenitor cell is equivalent to or higher than that of HUVEC, indicating that the cord blood-derived vascular endothelial progenitor cell is an effective candidate for a regenerative therapeutic agent.

Meanwhile, the present disclosure is characterized by a cell therapy composition for preventing or treating ischemic disease comprising the cord blood-derived vascular endothelial progenitor cell as an effective ingredient.

The cell therapy agent is a medicine (US FDA regulation) used for the purpose of treatment, diagnosis, and prevention by cells and tissues isolated, cultured, and manufactured through special manipulation from humans and animals, and refers to a medicine used for the purpose of treatment, diagnosis, and prevention through a series of actions such as proliferating and selecting living autologous, allogeneic, or xenogeneic cells in vitro or changing the biological characteristics of cells by other methods to restore the function of cells or tissues.

The cell therapy agent may additionally include a pharmaceutically acceptable carrier. The term "pharmaceutically acceptable" means that it is not toxic to cells or humans exposed to the composition. The carrier may be used without limitation so long as it is known in the art, such as a buffer, preservative, analgesic, solubilizer, isotonic agent, stabilizer, carrier, excipient, lubricant, or preservative.

The cell therapy agent may be manufactured in the form of various formulations according to commonly used techniques, and may be administered through any route so long as it may induce movement to the disease site. In some cases, it may be considered to load vascular endothelial progenitor cells into the vehicle equipped with a means for directing them to the lesion. Accordingly, the composition of the present disclosure may be administered through various routes, including topical (including buccal, sublingual, cutaneous and intraocular administration), parenteral (including subcutaneous, intradermal, intramuscular, drip, intravenous, intraarterial, intraarticular and intra cerebrospinal fluid) or transdermal administration.

The ischemic disease refers to a disease caused by a decrease in blood supply to a body organ, tissue or site caused by constriction or occlusion of blood vessels. After ischemia of the tissue or site, even in the case that reperfusion of blood occurs, nerve cells are damaged, causing various aftereffects, and ultimately leading to irreversible damage, that is, necrosis of cells and tissues. The ischemic disease may be selected from the group consisting of ischemic heart disease, ischemic myocardial infarction, ischemic heart failure, ischemic enteritis, ischemic vascular disease, ischemic eye disease, ischemic retinopathy, ischemic glaucoma, ischemic renal failure, ischemic baldness, ischemic stroke and ischemic lower limb disease, and more preferably, may be selected from the group consisting of ischemic heart disease, ischemic myocardial infarction, ischemic heart failure, ischemic enteritis, ischemic vascular disease, ischemic stroke and ischemic lower limb disease, and most preferably, may be ischemic myocardial infarction or ischemic lower limb disease.

Meanwhile, the present disclosure is also characterized by providing a method for obtaining a cord blood-derived vascular endothelial progenitor cell including:
treating cord blood with heparin to isolate and obtain monocytes from the cord blood; and
culturing the monocytes in a medium containing fucoidan, oleuropein, and vascular endothelial growth factor as effective ingredients to obtain cord blood-derived vascular endothelial progenitor cell. The medium may additionally contain 1 to 10% of human serum.

The cord blood may be supplied in a limited amount from the mother, and the amount of cord blood to be processed may be 1 to 1000 ml, preferably 10 to 100 ml, and more preferably 30 to 70 ml.

The heparin may be treated at a concentration of 1 to 1000 U per 1 ml of cord blood, preferably 5 to 100 U per 1 ml of cord blood, and most preferably 10 to 50 U per 1 ml of cord blood.

The treatment of the heparin may be performed at 1 to 20°C for 1 to 8 hours, preferably at 3 to 15°C for 2 to 6 hours, and most preferably at 4 to 10°C for 3 to 4 hours.

The heparin may be used by diluting it in physiological saline or DPBS (Dulbecco's phosphate-buffered saline), and it is preferable to treat the cord blood within 1 month from the start of using the heparin, and in the case that the treatment is performed on the cord blood after 3 months from the start of using, there is a problem that colonies of vascular endothelial progenitor cells are not secured after culture. In this specification, the start of use means the time when heparin is first exposed to the external environment and becomes usable, which is generally the same as the day when the heparin is opened from the storage container or packaging.

After the heparin treatment of the above cord blood, monocytes are separated and obtained. Monocytes may be obtained by density gradient centrifugation using ficoll.

The separated monocytes may be cultured in a medium containing fucoidan, oleuropein, and vascular endothelial growth factor as effective ingredients to obtain a cord blood-derived vascular endothelial progenitor cell. The medium may preferably additionally contain 1 to 10% of human serum.

The fucoidan may be included in the medium at a concentration of 0.05 to 20 µg/ml, preferably 0.1 to 10 µg/ml, and more preferably 0.1 to 5 µg/ml.

In addition, the oleuropein may be included in the medium at a concentration of 0.01 to 10 µM, preferably at a concentration of 0.1 to 5 µM, more preferably at a concentration of 0.2 to 0.75 µM.

In addition, the vascular endothelial growth factor may be included in the medium at a concentration of 1 to 500 ng/ml, preferably at a concentration of 10 to 300 ng/ml, more preferably at a concentration of 10 to 100 ng/ml.

In addition, the human serum may be included in the medium at a concentration of 1 to 10%, preferably at a concentration of 1 to 5%, more preferably at a concentration of 1 to 3%.

The medium refers to a culture solution that may support stem cell growth and survival under in vitro culture conditions, and includes all common media used in the art suitable for stem cell culture. The culture solution is a cell culture minimum medium (CCMM), and generally includes carbon source, nitrogen source, and trace element components. For example, ECGM (Endothelial Cell Growth Medium), DMEM (Dulbecco's Modified Eagle's Medium), MEM (Minimal essential Medium), BME (Basal Medium Eagle), RPMI1640, F-10, F-12, MEM (Minimal essential Medium), GMEM (Glasgow's Minimal essential Medium), Iscove's Modified Dulbecco's Medium, and the like may be used, and may include an endothelial cell growth medium, but is not necessarily limited thereto.

The culture may be performed in a well-plate coated with fibronectin at a concentration of 0.5 to 6 µg/cm², preferably at a concentration of 0.5 to 5 µg/cm².

Colonies of the cultured cells may be obtained and passaged to finally obtain a high-purity cord blood-derived vascular endothelial progenitor cell. At this time, the cord blood-derived vascular endothelial progenitor cell expressing the CD34 marker among the vascular endothelial progenitor cells may be passaged to finally obtain the high-purity cord blood-derived vascular endothelial progenitor cell.

At this time, the colonies attached to the fibronectin, and the like may be separated the cells using a protein decomposition enzyme reagent containing 1 to 5 mM EDTA, preferably a protein decomposition enzyme reagent containing 1.5 to 2 mM EDTA, and passaged to proceed with the cells. The EDTA chelates calcium and magnesium ions, thereby weakening intercellular and cell-matrix bonds, thereby helping the efficient functioning of protein-decomposing enzymes.

Hereinafter, the present disclosure will be described in detail with examples and experimental examples to specifically describe the present disclosure. However, the examples according to the present disclosure may be modified in various different forms, and the scope of the present disclosure should not be construed as being limited to the examples described below. The examples of the present disclosure are provided to more completely explain the present disclosure to a person having average knowledge in the art.

### Experimental Example 1: Identification of the number of colonies by heparin treatment

50 ml of cord blood of a pregnant woman (YBG-2022-012) is added at a concentration of 20 U of heparin per 1 ml of cord blood, reacted at 5°C for 4 hours, and then monocytes are obtained by density gradient centrifugation using ficoll.

The monocytes are obtained and seeded (1.8 ~ 2.0 x 10⁷ cells/well) in a 25T flask coated with fibronectin (2.5 µg/cm²), and cultured for 5 days in a medium containing 0.1 µg/ml fucoidan, 0.5 µM oleuropein, 100 ng/ml vascular endothelial growth factor, and human serum (2%). Next, colonies attached to fibronectin, and the like are separated using a proteolytic enzyme reagent containing 2 mM EDTA, and passage culture was performed, and the number of colonies is identified after 12 days (10 passages or more).

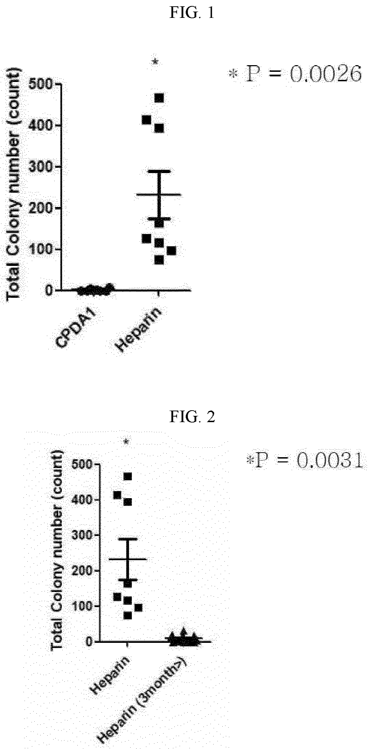

The number of colonies is compared with that of a comparative example that underwent the same culture process after treating CPDA-1, known as an anticoagulant, at the same concentration as heparin instead of the heparin, and this is shown in FIG. 1.

As shown in FIG. 1, it is identified that colonies of vascular endothelial progenitor cells can hardly be obtained from cord blood and monocyte cells that interacted with CPDA-1, and that at least 50 times more colonies can be obtained when heparin is treated under specific conditions.

### Experimental Example 2: Identification of the number of colonies according to heparin treatment conditions

Except for changing the temperature and reaction time of heparin as shown in Table 1 below, the experiment is conducted in the same manner as Experimental Example 1, and the final number of colonies generated is identified.

**[Table 1]**

| | | | | |
|---|---|---|---|---|
| Reaction temperature (°C) | 10 | 10 | 23 | 23 |
| Reaction time (hour) | 4 | 8 | 4 | 8 |
| Number of colonies | 28 | 9.3 | 1.25 | 3 |

The result of the experiment shows that when the reaction temperature and reaction time are different from those of Experimental Example 1, the number of colonies generated is relatively small.

### Experimental Example 3: Identification of the number of colonies according to the heparin dilution period

The culture is performed in the same manner as Experimental Example 1 except that the cord blood is treated after 3 months from the start of heparin use, and the results are shown in FIG. 2.

In general, the anticoagulant ability of heparin is maintained after 1 month or more of use. However, as in the experiment above, it is identified that almost no vascular endothelial progenitor cell colonies are formed after 3 months of use.

### Experimental Example 4: Surface antigen analysis of endothelial progenitor cells

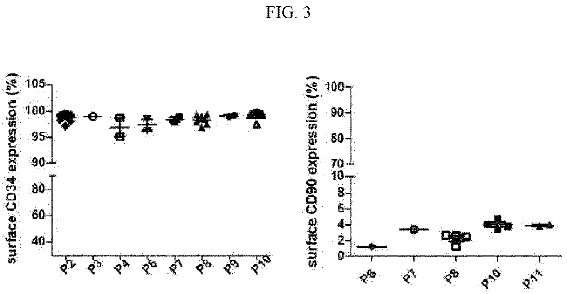

In Experimental Example 1, CD34 and CD90, which are surface antigens, are analyzed through antibody reaction using flow cytometry during passage culture, and the result is shown in FIG. 3.

As shown in FIG. 3, it is identified that at least 50% of the vascular endothelial progenitor cells expressed the CD34 marker, and at least less than 5% of the cell population expressed the CD90 marker.

CD34 positive cells exist at a level of 1.1 ± 0.9% in peripheral blood, 0.1 to 1% in cord blood, and 1.7 ± 0.5% in bone marrow, and there has been no report that late proliferative vascular endothelial progenitor cells show the CD34 positive cell ratio.

### Experimental Example 5: Obtaining vascular endothelial progenitor cells using a protein decomposition enzyme reagent

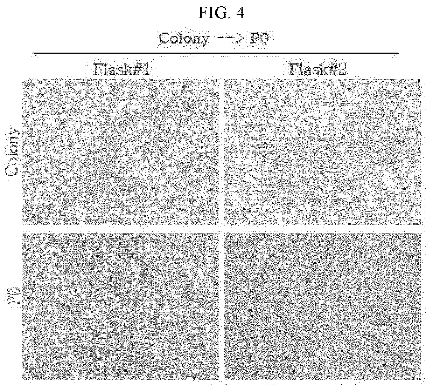

Instead of using the protein decomposition enzyme reagent containing 2 mM EDTA in Experimental Example 1, cell separation is performed using a TrypLE solution product containing 1 mM EDTA, and then passaging is performed. The result is shown in FIG. 4.

As shown in FIG. 4, when the Triple Solution product is used, cell separation is not performed normally, and MSC-like phenotype changes occurred in the cultured cells, and the changed cells does not regain their previous cell image.

### Experimental Example 6: Analysis of angiogenesis-stimulating factors of vascular endothelial progenitor cells

The vascular endothelial progenitor cells (XEPC), cord blood-derived mesenchymal stem cells (CBMSC), and fully differentiated vascular endothelial cells (HUVEC) cells obtained in Experimental Example 1 are cultured in 25T each and cultured in the same growth medium until 80% confluency, washed 2-3 times using serum-free basal media, and cultured for 3 days with serum-free media and the supernatant is recovered and centrifuged to remove debris, and then stored at -80°C.

The expression levels of ANGPT2, MCP1, MMP-1, and PIGF are quantitatively analyzed using the Angiogenesis Array Q1000 kit (Ray Biotech) for the samples, and the result is shown in Table 2 below.

**[Table 2]**

| Cell | ANGPT2 (pg/ml) | MCP1 (pg/ml) | MMP-1 (pg/ml) | PIGF (pg/ml) |
|---|---|---|---|---|
| XEPC | 135,000 | 4,300 | 15,200 | 2,300 |
| CBMSC | 3,100 | 1,300 | - | - |
| HUVEC | 5,000 | 3,800 | 14,600 | 2,150 |

The vascular endothelial progenitor cells (XEPC) obtained in Experimental Example 1 show a high expression level of ANGPT2, a signature gene of vascular endothelial progenitor cells, and it is identified that MCP1, MMP-1, and PIGF also showed expression levels equivalent to or higher than those of HUVEC.

### Experimental Example 7: Analysis of the effect of vascular endothelial progenitor cell injection in vivo

Diabetes is induced in nude mice by injecting vascular endothelial progenitor cells (STZ) obtained in Experimental Example 1, and after 1 month, femoral artery ligation is performed and Doppler imaging is performed to identify that blood flow is blocked.

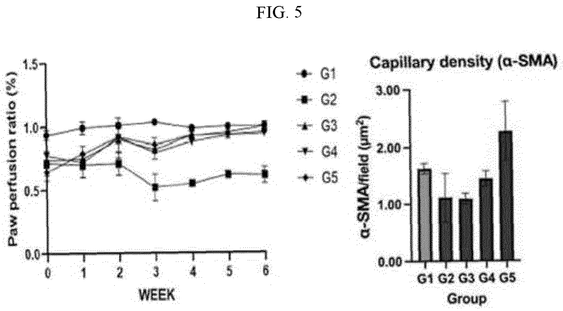

One day after ligation, the cell therapy agent containing the vascular endothelial progenitor cells of Experimental Example 1 is injected into the muscles on both sides of the injured blood vessel area, and blood flow is observed for 6 weeks. After autopsy, the density of new blood vessels per unit area is measured by IHC using α-SMA antibody, and the result is shown in FIG. 5.
G1, normal control group;
G2, disease-induced and untreated group;
G3, disease-induced and low-concentration cell therapy agent-treated group (0.5 x 10⁴ cells/head, n=5);
G4, disease-induced and medium-concentration cell therapy agent-treated group (1 x 10⁵ cells/head, n=5);
G5, disease-inducing and high-concentration cell therapy treatment group (5 x 10⁵ cells/head, n=5)

As shown in FIG. 5, the blood flow observation results show that the test substance administration group (G3-G5) showed no difference in blood flow until the first week compared to the untreated group (G2), but an improvement in blood flow is observed from the third week.

In addition, when examining the density of neovascularization per unit area by IHC using α-SMA antibody, it is identified that the test substance administration groups (G3-G5) show a tendency for neovascularization to increase depending on the concentration of cell therapy treatment.

## Claims

1. A cord blood-derived vascular endothelial progenitor cell,
wherein at least 50% or more of the cell population expresses CD34 marker, and
wherein at least 5% or less of the cell population expresses CD90 marker.

2. The cord blood-derived vascular endothelial progenitor cell according to claim 1,
wherein at least 80% or more of the cell population expresses the CD34 marker.

3. The cord blood-derived vascular endothelial progenitor cell according to claim 1,
wherein at least 80% or more of the cell population expresses CD144 marker and the CD184 marker.

4. The cord blood-derived vascular endothelial progenitor cell according to claim 1 is cultured for at least 10 passages.

5. The cord blood-derived vascular endothelial progenitor cell according to claim 1,
wherein an expression of at least one angiogenesis-stimulating factor selected from ANGPT2, MCP-1, MMP-1, and PIGF is increased.

6. A cell therapy composition for the prevention or treatment of ischemic disease, comprising the cord blood-derived vascular endothelial progenitor cell of any one of claims 1 to 5 as an effective ingredient.

7. A method for obtaining a cord blood-derived vascular endothelial progenitor cell, comprising:
treating cord blood with heparin to isolate and obtain monocytes from the cord blood; and
culturing the monocytes in a medium containing fucoidan, oleuropein, and vascular endothelial growth factor as effective ingredients to obtain the cord blood-derived vascular endothelial progenitor cell.

8. The method for obtaining a cord blood-derived vascular endothelial progenitor cell according to claim 7,
wherein the heparin is treated at a concentration of 1 to 1000 U per 1 ml of cord blood.

9. The method for obtaining a cord blood-derived vascular endothelial progenitor cell according to claim 7,
wherein the heparin treatment is performed at 1 to 20° C for 1 to 8 hours.

10. The method for obtaining a cord blood-derived vascular endothelial progenitor cell according to claim 7, further comprising:
passaging the cord blood-derived vascular endothelial progenitor cell expressing CD34 marker among the obtained cord blood-derived vascular endothelial progenitor cells to finally obtain a high-purity umbilical cord blood-derived vascular endothelial progenitor cell.
